# EUROPEAN PATENT SPECIFICATION

(11) **EP 3 819 287 B1**
(45) Date of publication and mention of the grant of the patent: **20.09.2023**
(21) Application number: 20216224.4
(22) Date of filing: 18.04.2017
(51) Int. Cl.: C07C 215/54, A61K 31/137, A61P 29/00

(54) **AMORPHOUS TAPENTADOL PHOSPHATE**
AMORPHOUS-TAPENTADOLPHOSPHAT
PHOSPHATE TAPENTADOL AMORPHE

(30) Priority: 19.04.2016 EP 16166099; 19.04.2016 EP 16166101; 23.05.2016 EP 16170780; 23.05.2016 EP 16170781
(43) Date of publication of application: 12.05.2021
(62) Divisional of application: 19208228.7
(73) Proprietor: ratiopharm GmbH, 89079 Ulm (DE)
(72) Inventor: ALBRECHT, Wolfgang, 89075 Ulm (DE); GUSERLE, Richard, 89259 Kötz (DE); GEIER, Jens, 72534 Hayingen (DE)
(74) Representative: Ter Meer Steinmeister & Partner

(56) References cited:
- WO-A1-2012/051246

## Description

The IUPAC name of tapentadol is 3-[1*R*,2*R*)-(3-dimethylamino)-1-ethyl-2-methylpropyl]phenol. Tapentadol is represented by the following chemical structure according to Formula (I):

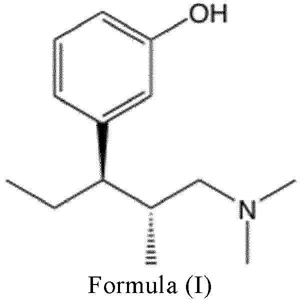

Tapentadol is an analgesic whose effect is reported to be based on two molecular mechanisms. Firstly, like opioids, tapentadol may activate µ-receptors and thus presynaptically and postsynaptically attenuates the transmission of pain stimuli in the spinal cord and brain. Secondly, tapentadol may act as a noradrenalin re-uptake inhibitor and thus increases the concentration of that nerve messenger in the synaptic gap.

Synthesis pathways for tapentadol and its use as an analgesic are described in EP 0 693 475 A1.

Further, tapentadol is reported to be present in form of addition salts, wherein for example WO 2006/000441 A2 describes tapentadol in form of its hydrochloride.

Further, WO 2012/010316 A1 relates to salts or co-crystals of tapentadol with at least one acid component. Said document refers to a long list of acid components, wherein among many others phosphoric acid is mentioned. However, no further information is given, neither to its method of preparation nor to its properties. Thus, the alleged tapentadol phosphate cannot be regarded as being enabled by the above-mentioned application.

WO 2012/051246 A1 substantially relates to the preparation of tapentadol hydrobromide. Further, in Example 8 said document inter alia describes the reaction of tapentadol free base with phosphoric acid, which resulted in tapentadol phosphate. The phosphate addition salt can be obtained as solid, which, however, is reported to liquefy within a few seconds. With respect to the workability and the regulatory requirements for pharmaceutical compositions and dosage forms such behaviour of the active pharmaceutical ingredient is highly undesirable.

With reference to WO 2012/010316 A1 and WO 2012/051246 A1 tapentadol phosphate was either regarded as not enabled or reported to liquefy within a few seconds. With respect to the workability and the regulatory requirements for pharmaceutical compositions and dosage forms such behaviour of the active pharmaceutical ingredient is highly undesirable.

Hence, it was an object of the present invention to overcome the above drawbacks.

According to the present invention, the above objectives can be achieved by amorphous tapentadol phosphate, which can advantageously be further processed in a pharmaceutical composition or a dosage form.

Thus, the present invention relates to amorphous tapentadol phosphate according to claim 1 and to a process for producing tapentadol phosphate in amorphous form. Further, the present invention relates to a composition comprising tapentadol phosphate and to a dosage form containing the composition of the invention.

In the context of this invention, the term "tapentadol phosphate" refers to the phosphoric acid addition salt of tapentadol according to Formula (II)

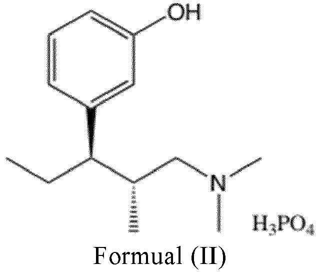

In addition, the term "tapentadol phosphate" as used in the present application can refer to tapentadol phosphate hydrates, solvates, polymorphs and mixtures thereof.

The term "amorphous" can be used in the context of this invention to designate the state of solid substances in which the components (atoms, ions or molecules) do not exhibit any periodic arrangement over a great range (= long-range order). In amorphous substances, the components are usually not arranged in a totally disordered fashion and completely randomly, but are rather distributed in such a way that a certain regularity and similarity to the crystalline state can be observed with regard to the distance from and orientation towards their closest neighbours (= short-range order). Amorphous substances consequently preferably possess a short-range order, but no long-range order.

In contrast to anisotropic crystals, solid amorphous substances are isotropic. Normally, they do not have a defined melting point but instead pass over into the liquid state after slowly softening. They can be distinguished from crystalline substances experimentally by means of X-ray diffraction, which does not reveal clearly defined interferences for them but rather, in most cases, only a few diffuse interferences with small diffraction angles.

A crystal form may be referred to herein as being characterized by data selected from two or more different data groupings, for example by a powder XRD pattern, having a group of specific peaks or by a powder XRD pattern as depicted in a diffractogram, or by "a combination thereof' (or "combinations thereof' or "any combination thereof'). These expressions, e.g. "any combination thereof', contemplate that the skilled person may characterize a crystal form using any combination of the recited characteristic analytical data. For example, the skilled person may characterize a crystal form using a group of three, four or five characteristic powder XRD peaks and supplement this characterization with one or more additional feature(s) observed in the powder X-ray diffractogram, e.g., an additional peak, a characteristic peak shape, a peak intensity or even the absence of a peak at some position in the powder XRD pattern. Alternatively, the skilled person may in some instances characterize a crystal form using a group of three, four or five characteristic powder XRD peaks and supplement that characterization with one or more additional feature(s) observed by using another analytical method, for example using one or more characteristic peaks in a solid state IR spectrum, solid state NMR or characteristics of the DSC thermogram of the crystal form that is being characterized.

In the present application, the XRPD is measured as described below in the experimental section. Further, unless indicated otherwise, XRPD peaks are reported as degrees 2θ values with a standard error of ± 0.2 degrees 2θ.

A crystal form may be referred to herein as being characterized by graphical data "as depicted in" a particular figure. Such data include for example powder X-ray diffractograms. The skilled person will understand that such graphical representation of data may be subject to small variations, e.g. in peak relative intensities and peak positions, due to factors such as variations in instrument response and variations in sample concentration and purity, which are well-known to the skilled person. Nonetheless, the skilled person would readily be capable of comparing the graphical data in the figures herein with graphical data generated for an unknown crystal form, and confirm as to whether the two sets of graphical data characterize the same crystal form or two different crystal forms.

Thus, in view of the above amorphous tapentadol phosphate can preferably be characterized by tapentadol phosphate of which the XRPD-pattern does not reveal clearly defined interferences, in particular does not reveal clearly defined interferences of tapentadol in form of its free base.

The amorphous tapentadol phosphate of the invention may consist of amorphous tapentadol phosphate. Alternatively, it may also contain small amounts of crystalline tapentadol phosphate components, provided that no interferences of crystalline tapentadol phosphate can be detected in an XRPD-pattern. A mixture containing 60 to 99.999% by weight amorphous tapentadol phosphate and 0.001 to 40% by weight crystalline tapentadol phosphate is preferred, more preferably 90 to 99.99% by weight amorphous tapentadol phosphate and 0.01 to 10% crystalline amorphous tapentadol phosphate, particularly preferably 95 to 99.9% by weight amorphous tapentadol phosphate and 0.1 to 5% crystalline tapentadol phosphate.

The tapentadol phosphate is present in combination with MHPO₄ or M'₂HPO₄, wherein M is a divalent cation and M' is a monovalent cation.

MHPO₄ or M'₂HPO₄, with M being a divalent cation and M' being a monovalent cation, can also refer to hydrates of MHPO₄ or M'₂HPO₄.

In a preferred embodiment monovalent cation M' can be a cation of an alkaline metal. Examples of alkaline metal are lithium, sodium and potassium.

In a more preferred embodiment the tapentadol phosphate is present in combination with MHPO₄, wherein M is a divalent cation. In a preferred embodiment divalent cation M' can be a cation of an alkaline earth metal. Examples of alkaline earth metals are magnesium or calcium. Especially preferred is calcium.

In a preferred embodiment the amorphous tapentadol phosphate of the present invention is present in solid form at 23°C. This includes that the amorphous tapentadol phosphate of the present invention does not liquefy or is not present in a solution.

In a further embodiment amorphous tapentadol phosphate can be present in isolated form. In the context of the invention an isolated form of amorphous tapentadol phosphate can be referred to as amorphous tapentadol phosphate being in essentially pure form. In particular, the amorphous tapentadol phosphate of the present invention can preferably not be present in combination with a carrier, for example in form of a solid solution.

A further subject of the present invention is a method of preparing amorphous tapentadol phosphate according to the present invention comprising the steps of
(aₐₘ) providing tapentadol and M(H₂PO₄)₂ or M'(H₂PO₄)
(bₐₘ) milling the mixture from step (aₐₘ)

In a preferred embodiment in step (aₐₘ) tapentadol and M(H₂PO₄)₂ or M'(H₂PO₄) can be provided. Tapentadol is preferably provided in form of its free base. In a particularly preferred embodiment tapentadol free base in polymorphic form B is provided. Tapentadol free base in polymorphic form B can for example be prepared as described in WO 2009/071310.

Generally, the comments made above for M or M' can also apply to the method of the present invention.

In a further preferred embodiment of the invention in step (aₐₘ) the molar ratio of tapentadol and M(H₂PO₄)₂ or M'(H₂PO₄) is from 2:1 to 1:1.7, preferably from 1.8:1 to 1:1.5, more preferably from 1.7: 1 to 1:1.

In a preferred embodiment of the method according to the present invention M(H₂PO₄)₂ or M'(H₂PO₄) can be present in the form of the corresponding monohydrate. In a particularly preferred embodiment M(H₂PO₄)₂ refers to Ca(H₂PO₄)₂xH₂O.

In a particularly preferred embodiment in step (aₐₘ) tapentadol free base in polymorphic form B and Ca(H₂PO₄)₂xH₂O are provided.

Further, step (aₐₘ) can comprise mixing tapentadol and M(H₂PO₄)₂ or M'(H₂PO₄).

In a further preferred embodiment of the method of the present invention in step (aₐₘ) or in step (bₐₘ) water is added, wherein the weight ratio of tapentadol (based on the weight of the free base) to water is from 50: 1 to 500:1, preferably from 75:1 to 400 :1.

In step (bₐₘ) the mixture from step (aₐₘ) can preferably be milled. The milling can preferably be performed in conventional milling apparatuses, such as in a ball mill, air jet mill, pin mill, classifier mill, cross beater mill, disk mill, mortar grinder or a rotor mill. A ball mill is preferably used. It is further preferred that 2 to 10 balls, preferably 3 to 7 balls, are used.

The milling time is preferably 0.5 minutes to 90 minutes, preferably 2 to 60 minutes, more preferably 5 to 30 minutes, in particular about 15 minutes.

In a further preferred embodiment of the method of the present invention in step (aₐₘ) water is added, wherein the weight ratio of tapentadol to water is from 50: 1 to 500:1, preferably from 75:1 to 400 :1.

It is preferred that the milled mixture can be sieved, preferably with a sieve having a mesh size of 1 to 1000 µm, preferably 20 to 800 µm, especially 25 to 600 µm.

The present invention furthermore relates to pharmaceutical compositions comprising amorphous tapentadol according to the present invention, wherein the pharmaceutical compositons additionally contain at least one pharmaceutically acceptable excipient.

As the pharmaceutically acceptable excipients, the proccessing of the pharmaceutical composition into an oral dosage form and the coating of a tablet are concerend, it applies the same as described above.

Pharmaceutically acceptable excipient(s) can for example be fillers, binders, glidants, disintegrants, lubricants, flow regulating agents and release agents. Suitable excipients are for example disclosed in "Lexikon der Hilfsstoffe für Pharmazie, Kosmetik und angrenzende Gebiete", published by H.P. Fielder, 4th Edition, and "Handbook of Pharmaceutical Excipients", 3rd Edition, published by A.H. Kibbe, American Pharmaceutical Association, Washington, USA, and Pharmaceutical Press, London.

The term filler generally means substances which serve to form the body of the tablet in the case of tablets with small amounts of active agent (e.g. less than 60% by weight). This means that fillers "dilute" the active agent(s) in order to produce an adequate tablet compression mixture. The normal purpose of fillers therefore is to obtain a suitable tablet size. Examples of preferred fillers are lactose, lactose derivatives, starch, starch derivatives, treated starch, chitin, cellulose and derivatives thereof, calcium phosphate, calcium hydrogen phosphate, sucrose, calcium carbonate, magnesium carbonate, magnesium oxide, maltodextrin, calcium sulphate, dextrates, dextrin and/or dextrose and hydrogenated vegetable oil. Fillers can be present in an amount of 0 to 80% by weight, preferably in an amount of 10 to 60% by weight based on the total weight of the composition.

A binder is generally a substance which is capable of increasing the strength of the resulting dosage form, especially the resulting tablets. Suitable binders are for example polyvinylpyrrolidone, hydroxypropyl cellulose, hydroxypropyl methylcellulose, methylcellulose, hydroxyethyl cellulose, sugars, dextran or corn starch. Binders can be present in an amount of 0 to 30% by weight, preferably in an amount of 2 to 15% by weight based on the total weight of the composition.

Glidants can be used to improve the flowability. Suitable glidants are for example alkaline earth metal salts of fatty acids, like stearic acid. The glidant can be present for example in an amount of 0 to 2% by weight, preferably in an amount of 0.5 to 1.5% by weight based on the total weight of the composition.

Disintegrants are compounds which enhance the ability of the dosage form, preferably the ability of the tablet, to break into smaller fragments when in contact with a liquid, preferably water. Suitable disintegrants are for example croscarmellose sodium, sodium carboxymethyl starch, cross-linked polyvinylpyrrolidone (crospovidone), sodium carboxymethylglycolate and sodium bicarbonate. The disintegrant can be present in an amount of 0 to 20% by weight, preferably in an amount of 1 to 15% by weight based on the total weight of the composition.

A suitable flow regulating agent is for example colloidal silica. The flow regulating agent can be present in an amount of 0 to 8% by weight, preferably in an amount of 0.1 to 3% by weight based on the total weight of the composition.

A suitable release agent is for example talcum. The release agent can be present in an amount of 0 to 5% by weight, preferably in an amount of 0.5 to 3% by weight based on the total weight of the composition. The parmaceutical composition can preferably be further processed into an oral doasage form, such as a capsule or tablet.

The oral dosage form, preferably a tablet or a capsule, more preferably a tablet, can preferably be coated, preferably film coated.

In the present invention the following three types of film coatings are possible:
- film coatings without affecting the release of the active ingredient,
- gastric juice-resistant film coatings,
- retard film coatings.

Generally, film coatings can be prepared by using film-forming agents, such as waxes, cellulose derivatives, poly(meth)acrylate, polyvinylpyrrolidone, polyvinyl acetate phthalate, and/or shellac or natural rubbers, such as carrageenan.

It is preferred that the present tablet is coated with a gastric juice-resistant film coating. Alternatively, a capsule comprising a gastric juice-resistant film coating can be used.

The gastric juice-resistant film coating preferably is a film coating being stable in the pH range of about 0.7 to 3.0, which is supposed to be the pH value of human gastric juice found in the stomach. However, in an environment with a pH value of 5 to 9, which is supposed to be present in the (small) intestine of the human body, the gastric juice-resistant film coating preferably dissolves and the drug can be released.

The gastric juice-resistant film coating (often also referred to as enteric coating) can comprise film-forming agents, for example fats, fatty acids, waxes, alginates, shellac, polyvinyl acetate phthalate, cellulose derivatives such as carboxy methyl ethyl cellulose, cellulose acetate succinate, cellulose acetate phthalate, hydroxypropyl methyl cellulose phthalate, hydroxypropyl methyl cellulose acetate succinate, cellulose acetate trimellitate, and meth(acrylic)acid copolymers, such as methyl acrylate-methacrylic acid copolymers, methyl methacrylate-methacrylic acid copolymers and Eudragits (for example Eudragit^{®} L30D, Eudragit^{®} L, Eudragit^{®} S).

The coating is preferably free of active ingredient. It is further preferred that the thickness of the coating is 10 µm to 2 mm, preferably 50 to 500 µm.

The preferred coating may comprise a film-forming agent and one or more of the following: lubricant, surfactant, glidant, pigment and water.

The preferred coating according to an embodiment of the present invention can comprise, along with the film-forming agent, e.g. stearic acid as lubricant for plasticizing and dissolving the polymer, sodium lauryl sulfate as a surfactant for wetting and dispersing, talc as glidant, iron oxide yellow and/or titanium oxide as pigment(s) and optionally purified water.

The present pharmaceutical composition and/or the oral dosage form of the present invention can be prepared by the methods well-known to a person skilled in the art, such as dry and wet granulation and direct compression.

In a preferred embodiment, the pharmaceutical composition and/or the oral dosage form can be administered one to three times a day, preferably once or twice a day, more preferably once a day.

The present invention further relates to amorphous tapentadol phosphate according to the present invention for use in the treatment of pain, preferably for use in the treatment of musculoskeletal pain.

Further, disclosed herein is a method of treating and/or preventing pain, preferably treating and/or preventing musculoskeletal pain, comprising administering to a subject in need thereof a therapeutically effective amount of amorphous tapentadol phosphate according to the present invention or the pharmaceutical composition according to the present invention.

### Experimental Part

### Analytical Methods

### X-ray Powder Diffraction (XRPD)

The samples were measured on a D8 Advance powder X-ray diffractometer (Bruker AXS, Karlsruhe, Germany) in a rotating PMMA sample holder (diameter: 25 mm; depth: 1 mm) in reflection mode (Bragg-Brentano geometry). Conditions of the measurements are summarized in the table below. Raw data were analyzed with the program EVA (Bruker AXS, Karlsruhe, Germany). Background subtraction and K*α*₂ stripping were not performed for the depicted diffractograms. Peak intensities were determined after background subtraction.

Conditions for powder diffraction measurements were as follows:

| | |
|---|---|
| Radiation | Cu K*α*₁/*α*₂ |
| Source | 34 kV / 40 mA |
| Detector | Vantec-1 (electronic window: 3°) |
| Kβ filter | Ni (diffracted beam) |
| measuring circle diameter | 435 mm |
| detector window slit | 12 mm |
| anti-scatter slit (diffracted beam) | 8 mm |
| divergence slit | v6.00 (variable) |
| Soller slit (incident /diffracted beam) | 2.5° |
| 20 range | 2° ≤ 2θ ≤ 55° |
| step size | 0.016 |
| step time | 0.2 s |

### Dynamic Vapor Sorption

Vapor sorption experiments were performed in the instrument SPSx-1µ (Projekt Messtechnik, Ulm, Germany) at 25°C and the humidity cycles as shown below.

| Cycle no. | rel. humidity (% RH) | | Number of steps | Time (h) | Comments |
|---|---|---|---|---|---|
| | start value | end value | | | |
| 1 | 40 | 0 | 4 | | |
| 2 | 0 | 5 | 1 | | |
| 3 | 5 | 75 | 7 | | |
| 4 | 75 | 75 | 0 | 24 h | To investigate the absorption of water at the upper humidity level during stress conditions |
| 5 | 75 | 95 | 2 | | |
| 6 | 95 | 90 | 1 | | |
| 7 | 90 | 0 | 9 | | |
| 8 | 0 | 5 | 1 | | |
| 9 | 5 | 35 | 3 | | |

### Thermogravimetry (TGA)

The samples were placed in open aluminum crucibles (40 µL). The measured weight curve is displayed as a function of the program temperature.

| | |
|---|---|
| Apparatus: | Mettler-Toledo TGA/DSC1 (Mettler-Toledo GmbH, Gießen, Germany) |
| Aluminium crucible: | 40 µL (open) |
| Temperature range: | 25°C to 400°C |
| Heating rate: | 10°C / min |
| Nitrogen flow: | 50 mL / min |
| Software: | STARe Version 11.00 |

### Examples:

### Reference Example 1:

### Preparation of tapentadol phosphate according to conventional methods:

### Reference Example 1a:

400 mg (2.1 mmol) tapentadol base were dissolved in 20 ml isopropanol and the solution was stirred at 23°C. After addition of 140 µl 85% phosphoric acid (2.1 mmol), the formation of a white precipitate was observed. Upon filtration of the suspension, the isolated solid spontaneously liquefied.

### Reference Example 1b:

530 mg Tapentadol base were dissolved in 10 ml isopropanol and the solution was stirred at 23°C. Upon addition of 160 µl 85% phosphoric acid, a white solid precipitated. 20 ml n-pentane were added and the suspension was filtered. The isolated solid liquefied within 2 min.

### Reference Example 1c:

150 µl 85% phosphoric acid (2.3 mmol) were dissolved in 4 ml water. 0.5 g (2.3 mmol) solid tapentadol base were added and the suspension was warmed to 50°C for 5 min until a clear solution was obtained. The solution was cooled to RT, then frozen in a bath of liquid nitrogen and lyophilized. Within two minutes after removal of the flask from the freeze dryer, the white solid liquefied.

### Reference Example 2:

### Preparation of crystalline Tapentadol phosphate with phosphoric acid:

In a 3 ml ball mill container, equipped with three 3 mm ZrO₂ balls, 200 mg Tapentadol base (Form B) and variable amounts of 85% phosphoric acid were mixed and milled for 15 min in the presence of 50 µl water. The products were isolated and analyzed by means of x-ray powder diffraction (XRPD).

The results are summarized below.

| **Exp.** | **H₃PO₄ 85%** | **Molar ratio (API** / **H₃PO₄)** | **water** | **result** |
|---|---|---|---|---|
| 1A | 61 µl | 1:1 | 50 µl | Crystalline tapentadol phosphate |
| 1B | 70 µl | 1: 1.15 | 50 µl | Crystalline tapentadol phosphate |

### Reference Example 2':

### Upscale preparation of crystalline tapentadol phosphate with phosphoric acid:

In a 20 ml ball mill container, equipped with twelve 3 mm ZrO₂ balls, 1 g tapentadol base (Form B) and 305 µl 85% phosphoric acid were mixed and milled for 15 min in the presence or absence of 250 µl water. The product was isolated and analyzed by means of x-ray powder diffraction (XRPD).

The result is summarized below.

| **Exp.** | **H₃PO₄ 85%** | **Molar ratio (Tap** / **H₃PO₄)** | **water** | **Result** |
|---|---|---|---|---|
| 1' | 1305 µl | 1:1 | 250 µl | Crystalline tapentadol phosphate |

### Reference Example 3:

### Preparation of crystalline tapentadol phosphate with sodium phosphate powder:

Tapentadol base (Form B) and Na(H₂PO₄)₂×2H₂O were given into a ball mill container together with three 3 mm ZrO₂ balls and water. These mixtures were treated in the ball mill for 15 min. The product was isolated and analyzed by means of x-ray powder diffraction (XRPD).

The result is summarized below.

| Exp. | Tapentadol base [mg] | NaH₂PO₄×2H₂O [mg] | Molar ratio | water | Result (XRPD) |
|---|---|---|---|---|---|
| 2 | 62.5 | 88.5 | 1:2 | 50 µl | Crystalline tapentadol phosphate |

### Reference Example 4.1:

### Preparation of crystalline tapentadol phosphate (type A)

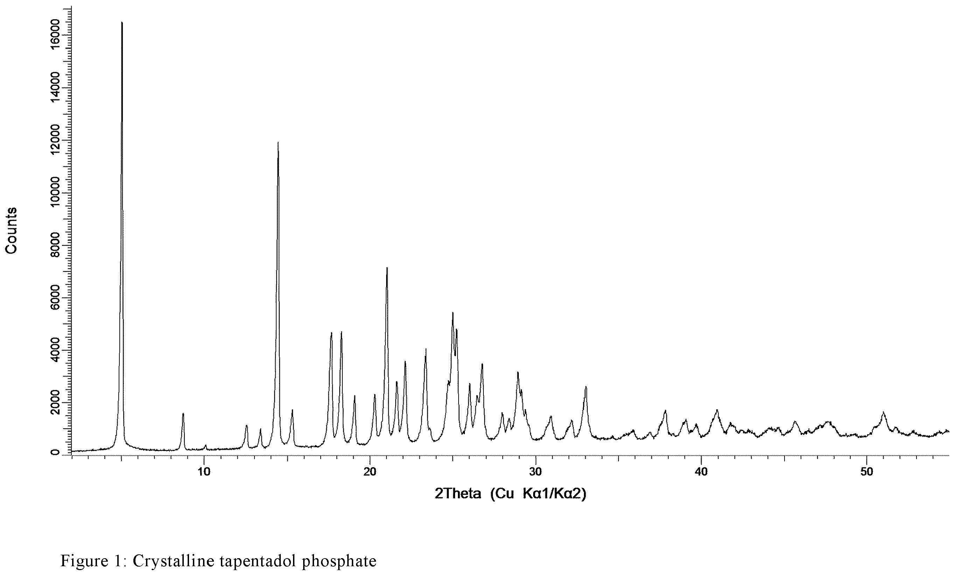

Tapentadol (1000 mg, 4.5 mmol) was dissolved at RT in a mixture of abs. ethanol (7.0 mL) and water (0.1 mL). Phosphoric acid 85% (0.31 mL; 4.5 mmol) was added under magnetic stirring at 23°C. The solution remained clear. After 15 minutes seed crystals of tapentadol phosphate were added and the mixture was stirred magnetically (200 rpm) at 23°C. Within one minute the crystallization started and after 10 min the formed suspension became too thick for stirring. It was stored at 23°C for 30 min. Cold (5°C) ethanol (20 mL) was added and the formed crystals were isolated immediately by vacuum filtration. The product was dried one hour in open atmosphere at 23°C (yield 976 mg).
- XRPD:: as shown in Figure 1

### Reference Example 4.2:

### Preparation of crystalline tapentadol phosphate

Tapentadol (1500 mg, 6.8 mmol) was dissolved at RT in abs. ethanol (10.5 mL). Phosphoric acid 85% (0.464 mL; 6.8 mmol) was added under magnetic stirring at 23°C. The solution remained clear. After 15 minutes seed crystals of tapentadol phosphate (7 mg) were added and the mixture was stirred magnetically (200 rpm) at 23°C. Within 10 minutes crystallization started and after 1h a thick suspension had been formed. It was stored at 23°C for 30 min. Cold (5-10°C) ethanol (20 mL) was added and the formed crystals were isolated immediately by vacuum filtration. The product was dried in open atmosphere at 23°C overnight. Yield: 550 mg. Weight loss up to 150°C (TGA): 2.3%.

### Reference Example 4.3:

### Preparation of crystalline tapentadol phosphate

Tapentadol (1000 mg, 4.5 mmol) was dissolved at RT in ethanol 96% v/v (7.0 mL). Phosphoric acid 85% (0.310 mL; 4.5 mmol) was added under magnetic stirring at 23°C. The solution remained clear. After 15 minutes seed crystals of tapentadol phosphate (5 mg) were added and the mixture was stirred magnetically (250 rpm) at 23°C. Within 5 minutes crystallization started and after 1 hour a thick suspension had been formed. It was stored at 23°C for 30 min. Cold (5-10°C) ethanol (20 mL) was added and the formed crystals were isolated immediately by vacuum filtration. The product was dried at 50°C / 9 mbar overnight. Yield: 933 mg. Weight loss up to 150°C (TGA): 1.7%.

### Reference Example 4.4:

### Preparation of crystalline tapentadol phosphate

Tapentadol (2.51 g) was dissolved at RT in a mixture of abs. ethanol (17.5 mL) and water (0.25 mL). Phosphoric acid 85% (0.773 mL) was added under magnetic stirring at 23°C. The solution remained clear. After 15 minutes seed crystals of tapentadol phosphate (12 mg) were added and the mixture was stirred magnetically (200 rpm) at 23°C. Within one minute crystallization started and after 10 min the formed suspension became too thick for stirring. It was stored at 23°C for 50 min. Cold (5°C) ethanol (32 mL) was added and the formed crystals were isolated immediately by vacuum filtration. The product was dried 16 h at 50°C / 9 mbar (yield 2.54 g). Weight loss up to 150°C (TGA): 2.6%.

### Reference Example 4.5:

### Preparation of crystalline tapentadol phosphate

Tapentadol (15.01 g) was dissolved at RT in a mixture of abs. ethanol (105 mL) and water (1.5 mL) and then heated at 50°C. Phosphoric acid 85% (0.55 mL) was added under magnetic stirring at 50°C. Seed crystals of tapentadol phosphate (0.75 g) were added, then additional phosphoric acid 85% (4.15 mL) was added dropwise at 50°C within 20 min. Crystallization started and the mixture was stirred 1 h at 50°C. It was cooled to 25°C within 45 min, then to 22°C. After stirring for 15 min at 22°C the product was isolated by vacuum filtration, washed with ethanol (40 mL) and dried at 50°C / 9 mbar for 16 h (yield 18.44 g). Weight loss up to 150°C (TGA): 2.0%.

### Reference Example 4.6:

### Preparation of crystalline tapentadol phosphate

Tapentadol (50.04 g) was dissolved at RT in a mixture of abs. ethanol (350 mL) and water (5 mL) and then heated at 50°C. Phosphoric acid 85% (1.75 mL) was added under magnetic stirring at 50°C. Seed crystals of tapentadol phosphate (1.5 g) were added, then additional phosphoric acid 85% (13.9 mL) was added dropwise at 50°C. The mixture was stirred 1 h at 50°C. Additional phosphoric acid 85% (0.75 mL) was added and the mixture was stirred overnight at 50°C. It was then cooled to 22°C within 60 min. After stirring for 2 h at 22°C the product was isolated by vacuum filtration, washed with ethanol (170 mL) and dried at 50°C / 9 mbar for 16 h (yield 49.50 g). Weight loss up to 150°C (TGA): 1.7%.

### Reference Example 4.7:

### Preparation of crystalline tapentadol phosphate

Tapentadol (446.4 g) was dissolved at RT in a mixture of abs. ethanol (3100 mL) and water (45 mL) and then heated at 50°C. Phosphoric acid 85% (15.8 mL) was added under stirring at 50°C. Seed crystals of tapentadol phosphate (7.8 g) were added, then additional phosphoric acid 85% (123 mL) was added dropwise at 50°C. The mixture was stirred overnight at 50°C. It was cooled to 22°C within 90 min. After stirring for 4 h at 22°C the product was isolated by vacuum filtration, washed with ethanol (1300 mL) and dried at 50°C / 9 mbar for 16 h (yield 485.1 g). Weight loss (TGA) up to 150°C: 2.6%.

### Reference Example 4.8:

### Preparation of crystalline tapentadol phosphate

Tapentadol (505.0 g) was dissolved at RT in a mixture of abs. ethanol (3530 mL) and water (50 mL) and then heated at 50°C. Phosphoric acid 85% (17.8 mL) was added under stirring at 50°C. Seed crystals of tapentadol phosphate (7.9 g) were added, then additional phosphoric acid 85% (138.5 mL) was added dropwise at 50°C. The mixture was stirred overnight at 50°C. It was cooled to 22°C within 60 min. After stirring for 4 h at 22°C the product was isolated by vacuum filtration, washed with ethanol (1400 mL) and dried at 50°C / 9 mbar for 16 h (yield 524.2 g). Weight loss up to 150°C (TGA): 2.4%.

### Reference Example 4.9:

### Preparation of crystalline tapentadol phosphate

Tapentadol (504.9 g) was dissolved at RT in a mixture of abs. ethanol (3520 mL) and water (50 mL). Phosphoric acid 85% (156 mL) was added within 10 min under stirring. The solution remained clear. Crystallization started after 20 min and the mixture became almost immobile after 45 min. It was slowly stirred for 3 h at RT. The suspension was diluted with cold (5 °C) ethanol (6700 mL) and the solid was isolated immediately by vacuum filtration. The product was dried at 50°C / 9 mbar for 16 h (yield 500.4 g). Weight loss up to 150°C (TGA): 2.2%.

### Reference Example 4.10:

### Preparation of crystalline tapentadol phosphate

Tapentadol (468.5 g) was dissolved at RT in a mixture of abs. ethanol (3200 mL) and water (47 mL). Phosphoric acid 85% (145 mL) was added within 10 min under stirring. The solution remained clear. Crystallization started after 20 min and the mixture became almost immobile after 45min. It was slowly stirred for 3h at RT. The suspension was diluted with cold (5 °C) ethanol (6000 mL) and the solid was isolated immediately by vacuum filtration. The product was dried at 50°C / 9 mbar for 16 h (yield 484.1 g). Weight loss up to 150°C (TGA): 2.5%.

### Reference Example 4.11:

### Preparation of crystalline tapentadol phosphate (type B)

200 mg of crystalline tapentadol phosphate type A obtained from Example 3.1 were heated at 70°C / 8 mbar for 12 h and tapentadol phosphate type B was obtained.

### Reference Example 4.12:

### Preparation of crystalline tapentadol phosphate (type B)

Tapentadol (10.00 g, type A) was stirred dissolved as a suspension in isopropanol (150 mL) at 70°C for 5 hours. The solid was isolated from the still warm suspension by vacuum filtration and dried at 23°C / 8 mbar for one hour (yield 8.80g). The product consisted of type B.

The table below shows selected X-ray powder diffractions peaks and water contents of **reference** examples 4.4 to 4.10

| Example | XRPD (I) | XRPD (II) | XRPD (III) | XRPD (IV) | XRPD (V) | Water content (%) |
|---|---|---|---|---|---|---|
| Ref 4.4 | 5.1 | 14.4 | 17.6 | 18.3 | 21.0 | 2.6 |
| Ref 4.5 | 5.2 | 14.5 | 17.8 | 18.5 | 21.2 | 2.0 |
| Ref 4.6 | 5.2 | 14.5 | 17.7 | 18.5 | 21.1 | 1.7 |
| Ref 4.7 | 5, .1 | 14.4 | 17.6 | 18.3 | 21.0 | 2.6 |
| Ref 4.8 | 5.1 | 14.4 | 17.6 | 18.3 | 21.0 | 2.4 |
| Ref 4.9 | 5.1 | 14.4 | 17.6 | 18.3 | 21.0 | 2.2 |
| Ref 4.10 | 5.1 | 14.4 | 17.7 | 18.3 | 21.0 | 2.5 |

From this table it can be seen that crystalline tapentadol phosphate can be obtained with different contents of water.

### Reference Example 5:

### Preparation of crystalline tapentadol phosphate with calcium phosphate powders (milling experiments):

Tapentadol base (Form B) and Ca(H₂PO₄)₂xH₂O was given into a ball mill container together with three 3 mm ZrO₂ balls and water. These mixtures were treated in the ball mill for 15 min. The products were isolated and analyzed by means of X-ray powder diffraction (XRPD). CaHPO₄×2H₂O was detected in all cases. Subsequently, the samples were subjected to a DVS cycle as described above. After a DVS treatment, additional crystalline reflections corresponding to tapentadol phosphate appeared.

| Exp. | Tapentadol base [mg] | Ca(H₂PO₄)₂xH₂O [mg] | Molar ratio | water | No. balls (size) / vol (container) | Result (XRPD) after DVS |
|---|---|---|---|---|---|---|
| Ref5 A | 300.4 | 300 | 1.14:1 | 200 µl | 12 (3 mm) / 20 ml | crystalline tapentadol phosphate |
| Ref 5B | 280.2 | 319.5 | 1.0:1 | 200 µl | 12 (3 mm) / 20 ml | crystalline tapentadol phosphate |
| Ref 5C | 360.1 | 240 | 1.70:1 | 200 µl | 12 (3 mm) / 20 ml | crystalline tapentadol phosphate |

The XRPD measurements of the examples revealed that crystalline tapendatol phosphate as depicted in Figure 1 was achieved.

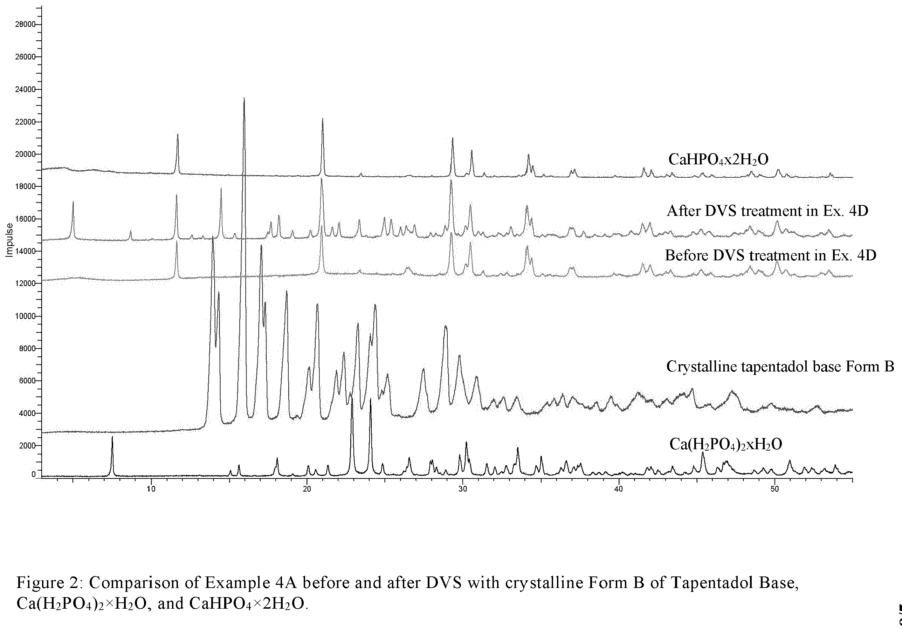

Further, it can be seen from Figure 2 that in Example 4 A after the step of subjecting the sample to a DVS the reflections of crystalline tapentadol phosphate appeared.

### Reference Example 6:

### Characterization

### 6.1 Base titration of tapentadol phosphate

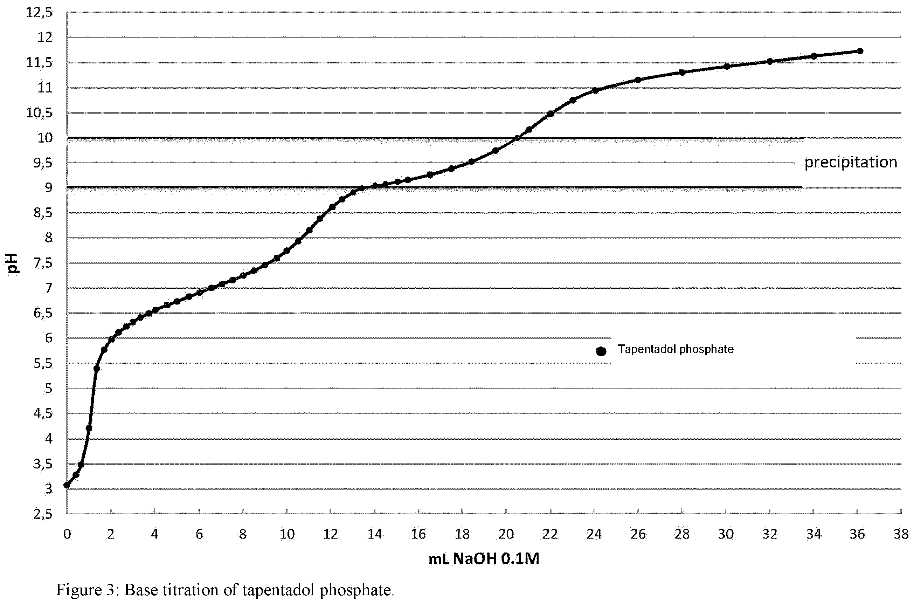

Tapentadol phosphate (300 mg) was dissolved in deionized water (40 mL). The solution was titrated at 23°C with 0.1M aqueous sodium hydroxide solution. The result is shown in Figure 3. The titration curve of tapentadol phosphate features three steps, corresponding to deprotonation of (H₂PO₄)⁻, (TAP-H)⁺ and (HPO₄)²⁻. Between pH 9 and pH 10 precipitation of tapentadol base is observed.

### 6.2. Water content

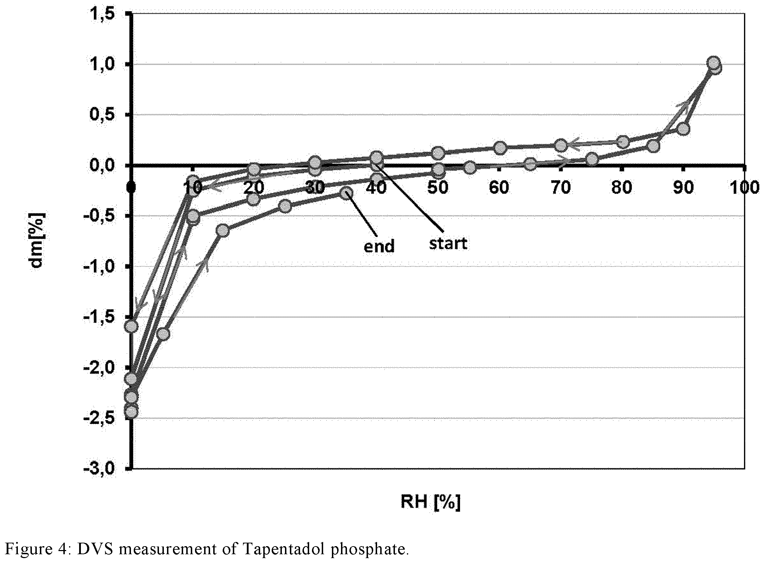

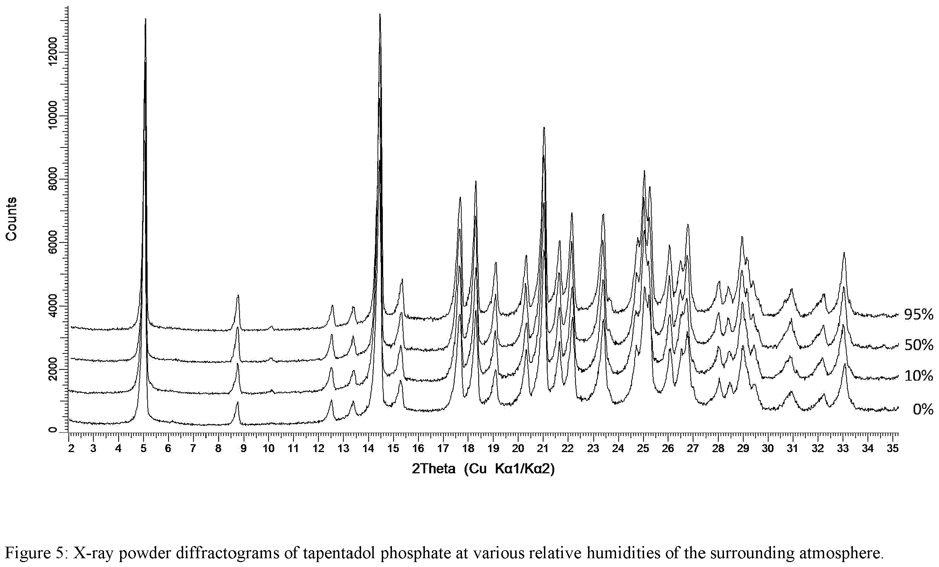

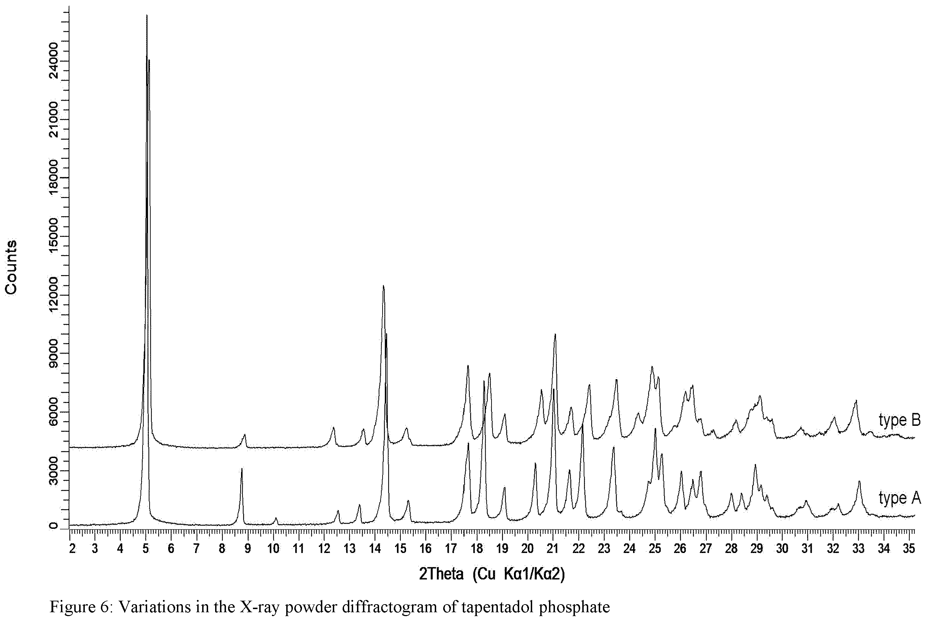

Tapentadol phosphate can contain water. TGA measurement indicated a water content of 2.6% (weight loss up to 150°C) for a sample prepared according to Ref Example 4.1 which had been further dried at 30°C / 8 mbar for 1 day (type A). Results of a dynamic vapor sorption measurement of this sample are shown in Figure 4. There is no pronounced variation in water content between 20 and 80% relative humidity (RH). At 0% RH a maximal weight loss of 2.5% is observed, in reasonable agreement with the TGA result. The theoretical water content for the stoichiometry of tapentadol phosphate hemihydrate is 2.74%. No changes were observed in the X-ray powder diffractograms of the sample before and after DVS measurement. Diffractograms measured from samples which had been equilibrated at 0, 10, 50, and 95% RH are practically identical, with exception of the peak at 10.1° (2Θ), which becomes weaker upon lowering of the relative humidity (Figure 5).

### 5.3 Molecular structure of crystalline tapentadol phosphate

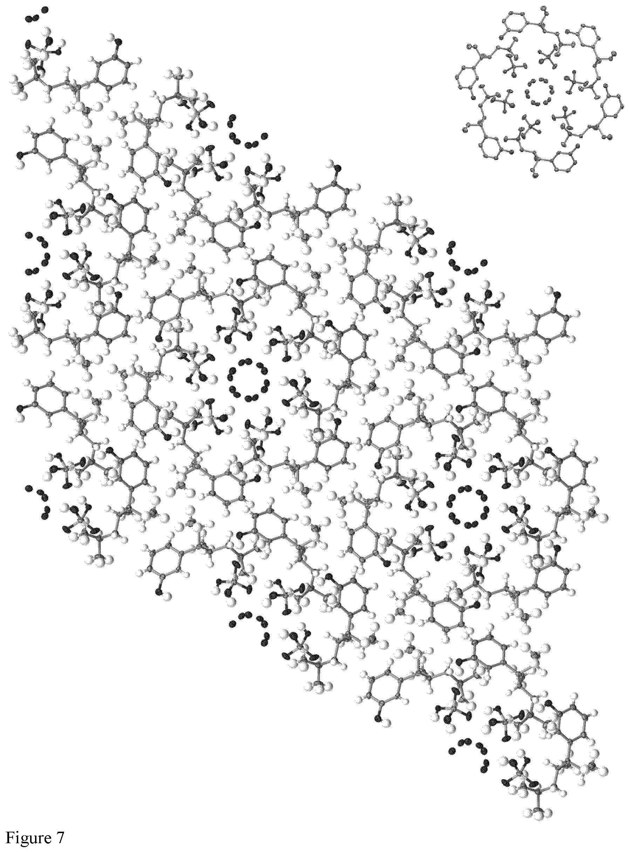

The crystal was measured on an Oxford Diffraction XCALIBUR2 diffractometer with area detector. The absolute configuration of the determined molecular structure matched the expected configuration; the Flack parameter was refined to a value of 0.10(12), thus it corroborates the assignment. Hydrogen atoms were refined according to a riding model (in case of the contained water molecules the hydrogen atoms were omitted). The structure is shown in Figure 7.

| | |
|---|---|
| Empirical formula | C₁₄H₂₆NO₆P |
| Formula weight | 335.34 |
| Temperature [K] | 150 |
| Wavelength [Å] | 0.71073 |
| Crystal system | hexagonal |
| Space group | *P*6₅ |
| Unit cell dimensions | |
| *a* [Å] | 20.2002(6) |
| *c* [Å] | 7.5599(2) |
| Volume [Å³] | 2671.52(7) |
| Z | 6 |
| Density (calculated) [g·cm⁻³] | 1.251 |
| Absorption coefficient [mm⁻¹] | 0.180 |
| F(000) | 1080 |
| Crystal size [mm] | 0.07 × 0.10 × 0.27 mm |
| Theta range for data collection [°] | 2.94 to 29.48 (reflection count complete at 25.05) |
| Index ranges | -26≤*h*≤27, -26<*k*<13, -10≤*l*≤7 |
| Reflections collected | 9740 |
| Independent reflections [R(int)] | 3713 (0.034) |
| Completeness to Theta = 25.05 | 98.9% |
| Absorption correction | Semi-empirical from equivalents |
| Max. and min. transmission | 0.88 and 1.00 |
| Refinement method | Full-matrix least-squares on *F*² |
| Data / parameters / restraints | 3712/222/8 |
| Goodness-of-fit on *F*² | 1.000 |
| Final R indices [*I*>2σ(*I*)] | *R*₁ = 0.0448, w*R*₂ = 0.0627 |
| Flack parameter | 0.10(12) |
| Final R indices [all data] | *R*₁ = 0.0529, w*R*₂ = 0.0663 |
| Largest diff. peak and hole [e·Å⁻³] | 0.36 and -0.35 |

### Experimental Part for amorphous tapentadol phosphate

### Analytical Methods

### X-ray Powder Diffraction (XRPD)

The samples were measured on a D8 Advance powder X-ray diffractometer (Bruker AXS, Karlsruhe, Germany) in a rotating PMMA sample holder (diameter: 25 mm; depth: 1 mm) in reflection mode (Bragg-Brentano geometry). Conditions of the measurements are summarized in the table below. Raw data were analyzed with the program EVA (Bruker AXS, Karlsruhe, Germany). Background subtraction and K*α*₂ stripping were not performed for the depicted diffractograms. Peak intensities were determined after background subtraction.

Conditions for powder diffraction measurements were as follows:

| | |
|---|---|
| Radiation | Cu K*α*₁/*α*₂ |
| Source | 34 kV / 40 mA |
| Detector | Vantec-1 (electronic window: 3°) |
| Kβ filter | Ni (diffracted beam) |
| measuring circle diameter | 435 mm |
| detector window slit | 12 mm |
| anti-scatter slit (diffracted beam) | 8 mm |
| divergence slit | v6.00 (variable) |
| Soller slit (incident /diffracted beam) | 2.5° |
| 20 range | 2° ≤ 2θ ≤ 55° |
| step size | 0.016 |
| step time | 0.2 s |

### Reference Example 1ₐₘ:

### Preparation of tapentadol phosphate according to conventional methods:

### Reference Example 1aₐₘ:

400 mg (2.1 mmol) tapentadol base were dissolved in 20 ml isopropanol and the solution was stirred at 23°C. After addition of 140 µl 85% phosphoric acid (2.1 mmol), the formation of a white precipitate was observed. Upon filtration of the suspension, the isolated solid spontaneously liquefied.

### Reference Example 1bₐₘ:

530 mg tapentadol base were dissolved in 10 ml isopropanol and the solution was stirred at 23°C. Upon addition of 160 µl 85% phosphoric acid, a white solid precipitated. 20 ml n-pentane were added and the suspension was filtered. The isolated solid liquefied within 2 min.

### Reference Example 1cₐₘ:

150 µl 85% phosphoric acid (2.3 mmol) were dissolved in 4 ml water. 0.5 g (2.3 mmol) solid tapentadol base were added and the suspension was warmed to 50°C for 5 min until a clear solution was obtained. The solution was cooled to RT, then frozen in a bath of liquid nitrogen and lyophilized. Within two minutes after removal of the flask from the freeze dryer, the white solid liquefied.

### Example 1ₐₘ:

### Preparation of powdery tapentadol phosphate

75 mg Tapentadol base (Form B) and 75 mg of Ca(H₂PO₄)₂×H₂O were given into a 3 ml ball mill container together with three 3 mm ZrO₂ balls and 50 µl of water. The mixture was treated in the ball mill for 15 min. The product was isolated and analyzed by means of X-ray powder diffraction (XRPD).

The results are summarized below. The identification of crystalline CaHPO₄ × 2H₂O indicated that tapentadol base may have converted into the corresponding amorphous phosphate salt.

| API | Excipient | Solvent (V=50 µl) | Result (XRPD) |
|---|---|---|---|
| TAPENTADOL | Ca(H₂PO₄)₂×H₂O | Water | Amorphous Tapentadol phosphate and crystalline CaHPO₄ × 2 H₂O |

### Example 2ₐₘ:

### Preparation of powdery tapentadol phosphate (milling experiments):

Tapentadol base (Form B) and Ca(H₂PO₄)₂×H₂O was given into a ball mill container together with three 3 mm ZrO₂ balls and water. These mixtures were treated in the ball mill for 15 min. The products were isolated and analyzed by means of X-ray powder diffraction (XRPD). The results are summarized below.

| Exp. | Tapentadol base [mg] | Ca(H₂PO₄)₂ ×H₂O [mg] | Molar ratio | water | No. balls (size) / vol. (container) | Result (XRPD) |
|---|---|---|---|---|---|---|
| 2Aₐₘ | 75.2 | 75 | 1.14:1 | 20 µl | 3 (3 mm) / 3 ml | CaHPO₄×2H₂O |
| 2Bₐₘ | 90.5 | 60 | 1.71:1 | 50 µl | 3 (3 mm) / 3 ml | CaHPO₄×2H₂O |
| 2Cₐₘ | 70.2 | 79.9 | 1.0:1 | 50 µl | 3 (3 mm) / 3 ml | CaHPO₄×2H₂O |
| 2Dₐₘ | 300.4 | 300 | 1.14:1 | 200 µl | 12 (3 mm) / 20 ml | CaHPO₄×2H₂O |
| 2Eₐₘ | 280.2 | 319.5 | 1.0:1 | 200 µl | 12 (3 mm) / 20 ml | CaHPO₄×2H₂O |
| 2Fₐₘ | 360.1 | 240 | 1.70:1 | 200 µl | 12 (3 mm) / 20 ml | CaHPO₄×2H₂O |

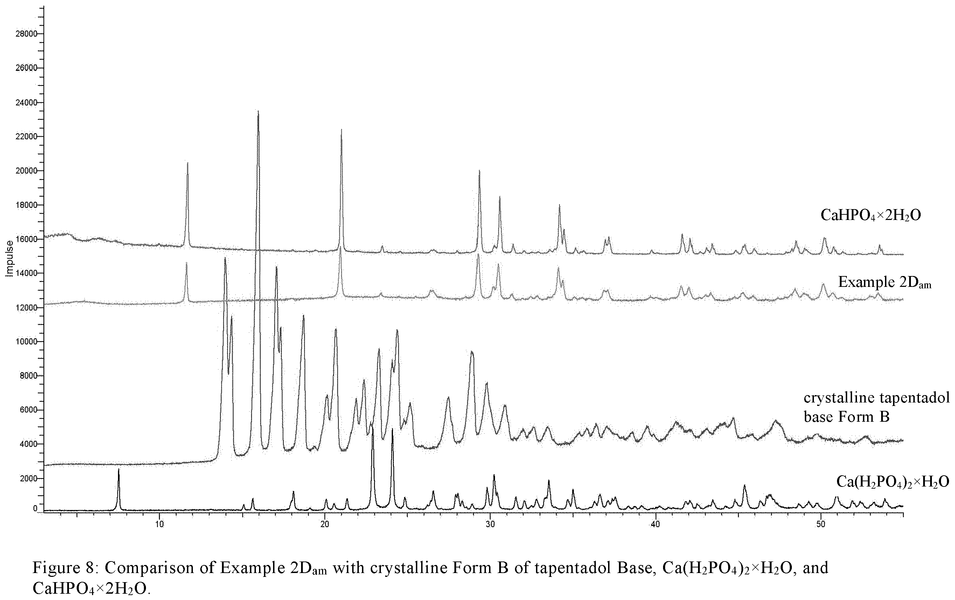

Further, with reference to Figure 8 the diffractogram of Example 2 Dₐₘ shows only reflections of crystalline CaHPO₄×2H₂O and none of tapentadol in form of its free base. Thus, considering the educts and the corresponding stoichiometry, tapentadol is converted to amorphous tapentadol phosphate.

## Claims

1. Amorphous tapentadol phosphate in combination with MHPO₄ or M'₂HPO₄, wherein M is a divalent cation and M' is a monovalent cation.

2. Amorphous tapentadol phosphate according to claim 1, wherein M is an alkaline earth metal cation.

3. Amorphous tapentadol phosphate according to claim 1 or 2, wherein M is a calcium cation.

4. Amorphous tapentadol phosphate according to any one of claims 1 to 3, being present isolated form.

5. Method for the preparation of amorphous tapentadol phosphate according to any one of claims 1 to 4 comprising the steps of
(aₐₘ) providing tapentadol and M(H₂PO₄)₂ or M'(H₂PO₄)
(bₐₘ) milling the mixture from step (aₐₘ)

6. Method according to claim 5, wherein in step (aₐₘ) the molar ratio of tapentadol to M(H₂PO₄)₂ or M'(H₂PO₄) is from 2:1 to 1:2.

7. Method according to claim 5 or 6, wherein M(H₂PO₄)₂ or M'(H₂PO₄) are present in form of the monohydrate.

8. Method according to any one of claims 5 to 7, wherein in step (aₐₘ) or (bₐₘ) water is added, wherein the weight ratio of tapentadol to water is from 50:1 to 500:1.

9. Pharmaceutical composition comprising amorphous tapentadol phosphate according to any one of claims 1 to 4 and further at least one pharmaceutically acceptable excipient.

10. Amorphous tapentadol phosphate according to any one of claims 1 to 4 for use in the treatment of pain.

## Patentansprüche

1. Amorphes Tapentadolphosphat in Kombination mit MHPO₄ oder M'₂HPO₄, wobei M ein zweiwertiges Kation und M' ein einwertiges Kation ist.

2. Amorphes Tapentadolphosphat nach Anspruch 1, wobei M ein Erdalkalimetallkation ist.

3. Amorphes Tapentadolphosphat nach Anspruch 1 oder 2, wobei M ein Calciumkation ist.

4. Amorphes Tapentadolphosphat nach einem der Ansprüche 1 bis 3, welches in isolierter Form vorliegt.

5. Verfahren zur Herstellung von amorphem Tapentadolphosphat nach einem der Ansprüche 1 bis 4, umfassend die Schritte
(aₐₘ) Bereitstellen von Tapentadol und M(H₂PO₄)₂ oder M'(H₂PO₄)
(bₐₘ) Mahlen der Mischung aus Schritt (aₐₘ)

6. Verfahren nach Anspruch 5, wobei in Schritt (aₐₘ) das Molverhältnis von Tapentadol zu M(H₂PO₄)₂ oder M'(H₂PO₄) von 2:1 bis 1:2 beträgt.

7. Verfahren nach Anspruch 5 oder 6, wobei M(H₂PO₄)₂ oder M'(H₂PO₄) in Form des Monohydrats vorliegen.

8. Verfahren nach einem der Ansprüche 5 bis 7, wobei in Schritt (aₐₘ) oder (bₐₘ) Wasser zugegeben wird, wobei das Gewichtsverhältnis von Tapentadol zu Wasser von 50:1 bis 500:1 beträgt.

9. Pharmazeutische Zusammensetzung, umfassend amorphes Tapentadolphosphat nach einem der Ansprüche 1 bis 4 und ferner mindestens einen pharmazeutisch verträglichen Hilfsstoff.

10. Amorphes Tapentadolphosphat nach einem der Ansprüche 1 bis 4 zur Verwendung bei der Behandlung von Schmerzen.

## Revendications

1. Phosphate de tapentadol amorphe en combinaison avec MHPO₄ ou M'₂HPO₄, dans lequel M est un cation divalent et M' est un cation monovalent.

2. Phosphate de tapentadol amorphe selon la revendication 1, dans lequel M est un cation métallique alcalino-terreux.

3. Phosphate de tapentadol amorphe selon la revendication 1 ou 2, dans lequel M est un cation calcium.

4. Phosphate de tapentadol amorphe selon l'une quelconque des revendications 1 à 3, étant présent sous une forme isolée.

5. Procédé pour la préparation de phosphate de tapentadol amorphe selon l'une quelconque des revendications 1 à 4, comportant les étapes consistant à :
(aₐₘ) fournir du tapentadol et du M(H₂PO₄)₂ ou du M'(H₂PO₄),
(bₐₘ) broyer le mélange de l'étape (aₐₘ).

6. Procédé selon la revendication 5, dans lequel à l'étape (aₐₘ), le rapport molaire du tapentadol sur le M(H₂PO₄)₂ ou le M'(H₂PO₄) est de 2:1 à 1:2.

7. Procédé selon la revendication 5 ou 6, dans lequel M(H₂PO₄)₂ ou M'(H₂PO₄) sont présents sous la forme du monohydrate.

8. Procédé selon l'une quelconque des revendications 5 à 7, dans lequel à l'étape (aₐₘ) ou (bₐₘ), de l'eau est ajoutée, dans lequel le rapport pondéral du tapentadol sur l'eau est de 50:1 à 500:1.

9. Composition pharmaceutique comportant du phosphate de tapentadol amorphe selon l'une quelconque des revendications 1 à 4 et en outre au moins un excipient acceptable du point de vue pharmaceutique.

10. Phosphate de tapentadol amorphe selon l'une quelconque des revendications 1 à 4 pour une utilisation dans le traitement d'une douleur.
